(19) Europäisches Patentamt
European Patent Office
Office européen des brevets

(11) Veröffentlichungsnummer: **0 231 888 B1**

(12) # EUROPÄISCHE PATENTSCHRIFT

(45) Veröffentlichungstag der Patentschrift: **30.12.92**

(51) Int. Cl.5: **C07D 239/48**, A61K 31/505

(21) Anmeldenummer: **87101246.4**

(22) Anmeldetag: **29.01.87**

(54) **Substituierte 2,4-Diamino-5-benzylpyrimidine, deren Herstellung und deren Verwendung als Arzneimittel mit antimikrobieller Wirksamkeit.**

(30) Priorität: **06.02.86 DE 3603577**

(43) Veröffentlichungstag der Anmeldung:
**12.08.87 Patentblatt 87/33**

(45) Bekanntmachung des Hinweises auf die Patenterteilung:
**30.12.92 Patentblatt 92/53**

(84) Benannte Vertragsstaaten:
**AT BE CH DE ES FR GB GR IT LI LU NL SE**

(56) Entgegenhaltungen:
**EP-A- 0 003 212**
**EP-A- 0 054 756**
**DE-A- 1 445 176**
**DE-A- 1 720 031**
**DE-A- 2 010 166**
**DE-A- 2 720 771**
**US-A- 4 258 045**

**JOURNAL OF MEDICINAL CHEMISTRY, Band 24, 1981, Seiten 933-941, American Chemical Society, Washington DC, US; B. ROTH et al.: "2,4-Diamino-5-benzylpyrimidines and analogues as antibacterial agents. 5. 3,5-Dimethoxy-4'-substituted-benzyl analogues of trimethoprim"**

(73) Patentinhaber: **Fatol Arzneimittel GmbH**
**Robert-Koch-Strasse Postfach 1260**
**W-6685 Schiffweiler(DE)**

(72) Erfinder: **Seydel, Joachim K., Prof.Dr.**
**Mühloh 2**
**W-2061 Borstel(DE)**
Erfinder: **Haller, Rolf, Prof.Dr.**
**Kronskamp 3**
**W-2300 Kiel(DE)**
Erfinder: **Kansy, Manfred,**
**Kantstrasse 17**
**W-2300 Kiel(DE)**
Erfinder: **Hachtel, Gerd**
**Römerweg 4**
**W.-2313 Raisdorf(DE)**

(74) Vertreter: **UEXKÜLL & STOLBERG Patentanwälte**
**Beselerstrasse 4**
**W-2000 Hamburg 52(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue substituierte 2,4-Diamino-5-benzylpyrimidine, deren Herstellung sowie deren Verwendung als Arzneimittel zur Behandlung mikrobieller und insbesondere mycobakterieller Infekte.

Die antibakterielle Wirksamkeit von Verbindungen des Benzylpyrimidin-Typs ist bekannt. In einer Reihe von Veröffentlichungen werden antibakteriell wirksame Verbindungen wie insbesondere 2,4-Dimino-5-benzyl-pyrimidine beschrieben (DE-OS 27 20 771, EP-A 0 003 212, EP-A 0 054 756, DE-OS 1 720 031, DE-OS 1 445 176 und B. Roth et al., Journal of Medicinal Chemistry, Bd. 24, S. 933-941 (1981).

In keiner der vorhergehenden Druckschriften werden jedoch die neusubstituierten 2,4-Diamino-5-benzyl-pyrimidine der vorliegenden Erfindung beschrieben.

Derartige Verbindungen, wie beispielsweise die bekannten Präparate Trimethoprim, Brodimoprin und Tetroxoprim entfalten ihre Wirksamkeit durch Hemmung der Dihydrofolsäure-Reduktase (DHFR) bei grammnegativen und grammpositiven Bakterien. Es zeigte sich jedoch, daß diese Verbindungen gegenüber Mycobakterien nur eine äußerst geringe Wirksamkeit aufweisen.Die zur Hemmung des Mycobakterien-Wachstumserforderlichen Konzentrationen dieser Mittel sind so hoch, daß sie in vivo nicht erreichbar bzw. nicht verträglich sind.

Aufgabe der Erfindung ist es demgemäß, Verbindungen zu schaffen, die bereits in niedriger physiologisch vertretbarer Konzentration wirkungsvolle Hemmstoffe mikrobiellen Wachstums und insbesondere des Mycobakterien-Wachstums sind.

Zur Lösung dieser Aufgabe werden die neuen substituierten 2,4-Diamino-5-benzylpyrimidine der allgemeinen Formel I

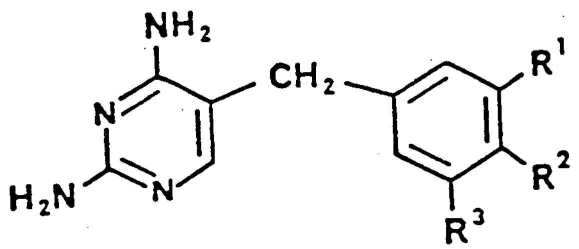

I

vorgeschlagen, in der einer der Substituenten $R^1$ bis $R^3$

eine 2',4'-substituierte Phenyl-4-sulfonylphenylaminoalkoxy-, Phenyl-4-sulfonylphenyl-aminoalkylthio-, Phenyl-4-sulfonylphenylalkoxy- oder Phenyl-4-sulfonylphenylalkylthiogruppe ist,

wobei die Substituenten in 2',4'-Stellung gleich oder verschieden und Wasserstoff, Amino-, Alkylamino-, Dialkylamino-, Alkoxy-, Alkyl-, Nitro-, Alkylthio- und/oder Acetaminogruppen sind,

und die beiden anderen der Substituenten $R^1$ bis $R^3$ gleich oder verschieden und Wasserstoff-, Alkoxy-, Alkylthio-, und/oder Alkylaminogruppen sind.

Der Alkylrest der beiden anderen Substituenten $R^1$ bis $R^3$ weist vorzugsweise 1 bis 3 C-Atome und insbesondere 1 C-Atom auf.

Bevorzugte Beispiele für die erfindungsgemäßen Verbindungen sind:

- 2,4-Diamino-5-[3,5-dimethoxy-4-(4'-nitrophenyl-4-sulfonylphenyl)methoxybenzyl]-pyrimidin (K 95)
- 2,4-Diamino-5-(3,5-dimethoxy-4-[3-(4'-nitrophenyl-4-sulfonylphenylamino)propoxy]benzyl)-pyrimidin (K 122)
- 2,4-Diamino-5-(4-methoxy-3-[2-(4'-nitrophenyl-4-sulfonylphenylamino)ethoxy]benzyl)-pyrimidin (K 132)
- 2,4-Diamino-5-(4-methoxy-3-[3-(4'-nitrophenyl-4-sulfonylphenylamino)propoxy]benzyl)-pyrimidin (K 135)
- 2,4-Diamino-5-[4-(4'-aminophenyl-4-sulfonylphenylmethoxy)-3,5-dimethoxybenzyl]-pyrimidin (K 96)
- 2,4-Diamino-5-(4-[3-(4'-aminophenyl-4-sulfonylphenylamino)-propoxy]-3,5-dimethoxybenzyl)-pyrimidin (K 130)
- 2,4-Diamino-5-[3-(4'-aminophenyl-4-sulfonylphenylmethoxy)-4-methoxybenzyl]-pyrimidin (K 128)
- 2,4-Diamino-5-(3-[2-(4'-aminophenyl-4-sulfonylphenylamino)ethoxy]-4-methoxybenzyl)-pyrimidin (K 138)
- 2,4-Diamino-5-(4-[3-(4'-aminophenyl-4-sulfonylphenylamino)propoxy]-3,5-dimethoxybenzyl)-pyrimidin (K 137)
- 2,4-Dimino-5-[4-(4'-aminophenyl-4-sulfonylphenylmethoxy)-3-methoxybenzyl]-pyrimidin (K 120)

- 2,4-Diamino-5-(4-[3-(4'methylphenyl-4-sulfonylphenylamino)propoxy]-3,5-dimethoxybenzyl)-pyrimidin (K 150)
- 2,4-Diamino-5-(4-[2-(4'-aminophenyl-4-sulfonylphenylamino)ethoxy]-3,5-dimethoxybenzyl)-pyrimidin (K 107)
- 2,4-Diamino-5-(4-[2-(2'-methyl-4'-aminophenyl-4-sulfonylphenylamino)ethoxy]-3,5-dimethoxybenzyl)-pyrimidin.

Von der Erfindung sind ferner die physiologisch verträglichen Säureaddionssalze der erfindungsgemäßen Verbindungen mit anorganischen oder organischen Säuren umfaßt.

Erfindungsgemäß zeigte sich überraschenderweise, daß die Einführung der Substituenten gemäß Anspruch 1 in Verbindungen vom Typ der Benzylpyrimidine zu einer dramatischen Steigerung der Wirksamkeit dieser Verbindung als Hemmstoffe des Mycobakterien-Wachstums führt (vgl. Tabelle 1).

Die erfindungsgemäßen substituierten 2,4-Diamino-5-benzylpyrimidine können hergestellt werden, indem man entweder

a) eine Verbindung der allgemeinen Formel II

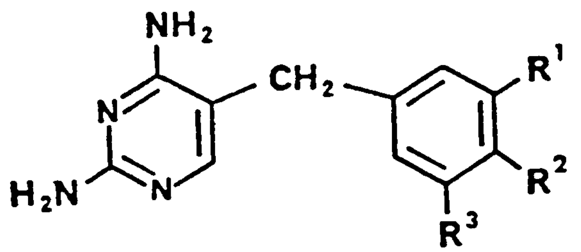

II ,

in der einer der Substituenten $R^1$ bis $R^3$ eine Hydroxyl- bzw. eine Mercaptogruppe ist und die beiden anderen der Substituenten $R^1$ bis $R^3$ gleich oder verschieden Wasserstoff-, Alkoxy-, Alkylthio- und/oder Alkylaminogruppen sind, mit einer Verbindung der allgemeinen Formeln III

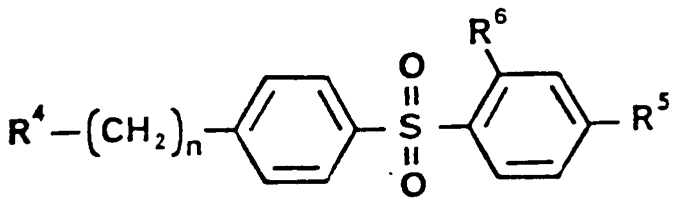

III

oder IV

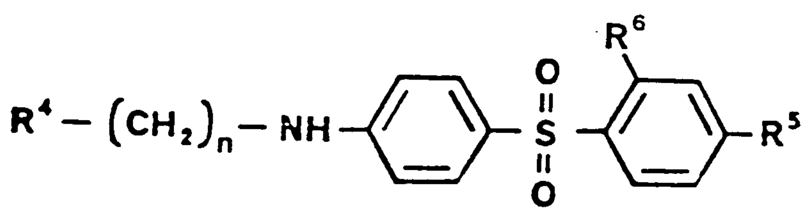

V

verethern, in denen $R^4$ ein Halogenrest ist und $R^5$ und $R^6$ gleich oder verschieden und Wasserstoff, Amino-, Alkylamino-, Dialkylamino-, Alkoxy-, Alkyl-, Nitro-, Alkylthio- und/oder Acetaminogruppen sind.

Die Veretherung wird zweckmäßigerweise in an sich bekannter Weise in Lösungsmitteln wie Wasser, Methanol, Ethanol, n- und Isopropanol, Butanol, Dimethylformamid, Dimethylsulfoxid, Aceton, Methylethylketon und mono- und dialkylierten Ethern des Ethylenglykols und Diethylenglykols und deren Gemischen unter Zusatz einer Base wie Natrium, Natriumethylat, Natrium- oder Kaliumhydroxid, Kalium- oder Natriumcarbonat bei einer Temperatur zwischen -20°C und der Siedetemperatur der verwendeten Lösungsmittel, vorzugsweise aber Raumtemperatur durchgeführt.

In einer bevorzugten Ausführungsform der Erfindung wird die Veretherung in Ethylenglykolmonomethylether als Lösungsmittel und dem Natriumalkoholat des Ethylenglykolmonomethylethers durchgeführt.

Ferner können die Verbindungen gemäß Anspruch 1 hergestellt werden, indem man

b) eine Verbindung der allgemeinen Formel V

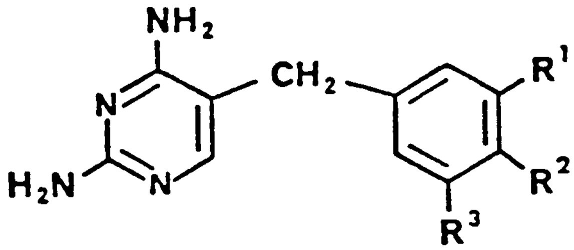

V,

reduziert, in der einer der Substituenten $R^1$ bis $R^3$ eine 2',4'-substituierte Phenyl-4-sulfonylphenylaminoalkoxy-, Phenyl-4-sulfonylphenylaminoalkylthio-, Phenyl-4-sulfonylphenylalkoxy- oder Phenyl-4-sulfonylalkylthiogruppe mit endständiger Nitrogruppe ist, wobei die Substituenten in 2',4'-Stellung gleich oder verschieden und Wasserstoff, Amino-, Alkylamino-, Dialkylamino-, Alkoxy-, Alkyl-, Nitro-, Alkylthio- und/oder Acetamino- und die beiden anderen der Substituenten $R^1$ bis $R^3$ gleich oder verschieden Wasserstoff-, Alkoxy-, Alkylthio- und/oder Alkylaminogruppen sind.

Die Reduktion wird zweckmäßigerweise in an sich bekannter Weise in Lösungsmitteln wie Wasser, Methanol, Ethanol, Eisessig, Essigsäureethylester, Dimethylformamid, Wasser/Ethanol, Wasser/Dioxan, Wasser/Tetrahydrofuran, Ethylenglykolmonomethylester und weiteren Alkyl- und Arylethern des Ethylenglykols, Diethylenglykols und deren Gemischen in Gegenwart eines Hydrierungskatalysators wie Raney Nickel, Platin oder Palladium/Kohle durchgeführt. Die Reduktion kann mit Metallen wie Eisen, Zinn oder Zink in Gegenwart einer Säure wie Salzsäure oder Essigsäure, mit Salzen wie Eisen(II)-sulfat, Zinn(II)-chlorid/Salzsäue oder Natriumdithionid in Gegenwart einer Base wie Natronlauge, Pyridin oder Hydrazin und Raney-Nickel durchgeführt werden, wobei die Temperaturen zwischen 0 und 100°C, vorzugsweise aber 50°C betragen.

Es hat sich als besonders vorteilhaft erwiesen, die Reduktion in Ethylenglykolmonomethylether, Methanol oder Gemischen derselben bei 50°C und 1 bis 6 bar in Gegenwart von Raney-Nickel W2 (hergestellt nach Org. Synth. Coll. 3, 181 ( 1955)) oder Palladium/Kohle durchzuführen.

Die Wirksamkeit der erfindungsgemäßen Verbindungen als Wachstumsinhibitoren für insbesondere Mycobakterien wurde an Ganzbakterienkulturen nachgewiesen, indem die minimalen Hemmkonzentrationen (MHK) und die für eine halbmaximale Wachstumshemmung ($I_{50}$) erforderlichen Konzentrationen bei Verwendung typischer Vertreter der erfindungsgemäßen Verbindungen ermittelt wurden.

Überraschenderweise zeigte sich dabei eine bis um das 300fache erhöhte Wirksamkeit der erfindungsgemäßen Verbindungen gegenüber handelsüblichen Präparaten wie beispielsweise Pyrimethamin. Zusätzlich wurde ein synergistischer Effekt von Kombinationen der beanspruchten Verbindungen mit Hemmstoffen der Dihydropteroinsäuresynthetase wie beispielsweise mit Diaminodiphenylsulfon (DDS) beobachtet.

Die erfindungsgemäßen Verbindungen sind demgemäß allein oder in Kombination mit Hemmstoffen vom Typ der Diaminodiphenylsulfone, Ring-substituierten 4-Aminodiphenylsulfone oder Ring- und/oder Stickstoff-substituierten Diaminodiphenylsulfone und/oder antibakteriell wirkenden Sulfonamide als Wirkstoffe in Arzneimitteln zur Behandlung mikrobieller Infektionen und insbesondere von Mycobakteriosen geeignet; ihre Bedeutung liegt unter anderem auch in der Möglichkeit, sowohl partiell DDS- als auch TMP-resistente Mycobakteriosen zu behandeln oder die Resistenzentwicklinng bei der DDS- oder TMP-Monotherapie zu vermeiden.

Die erfindungsgemäßen Arzneimittel enthalten die erwähnten Wirkstoffe bzw. Wirkstoffkombinationen zusammen mit einem verträglichen pharmazeutischen Träger. Dieser Träger kann ein für die enterale, perkutane oder parenterale Verabreichung geeignetes organisches oder anorganisches Trägermaterial sein, wie z.B. Wasser, Gummi arabicum, Lactose, Stärke, Magnesiumstearat, Talg, pflanzliche Öle, Polyalkylenglykole, Vaseline und dergleichen. Darüber hinaus können die Präparate weitere pharmazeutisch wirksame Stoffe enthalten, wie fiebersenkende Mittel, schmerzstillende Mittel, entzündungshemmende Mittel und dergleichen. Die pharmazeutischen Präparate können oral, beispielsweise in Form von Tabletten, Kapseln, Pillen, Pulvern, Granulaten, Lösungen, Sirupen, Suspensionen, Elixieren und dergleichen verabreicht werden. Die Verabreichung kann aber auch parenteral, z.B. in Form von sterilen Lösungen, Suspensionen oder Emulsionen oder lokal in Form von Lösungen, Suspensionen, Salben, Pudern, Aerosolen und dergleichen

erfolgen.

Ferner können die pharmazeutischen Präparate sterilisiert sein und/oder Bestandteile wie Konservierungsmittel, Stabilisatoren, Netzmittel, Emulgatoren, Salze und Puffersubstanzen enthalten.

Die Erfindung wird nachfolgend anhand von Beispielen erläutert.

## Beispiel 1

Herstellung von 2,4-Diamino-5-[3,5-dimethoxy-4-(4'-nitrophenyl-4-sulfonylphenyl)-methoxybenzyl]-pyrimidin (K 95)

2,76 g (0,01 Mol) 2,4-Diamino-5-(4-hydroxy-3,5-dimethoxybenzyl)-pyrimidin (J. med. Chem. 14 58 (1971) wurden durch Rühren in einer Lösung von 0,23 g (0,01 Mol) Natrium in 45 ml Ethylenglykolmonomethylether gelöst. Anschließend wurden 3,92 g (0,011 Mol) 4-Brommethyl-4'-nitrodiphenylsulfon (J. Chem. Soc. 22 1508 (1957)) zugegeben und 17 Stunden bei Raumtemperatur gerührt.

Das Endprodukt wurde durch Einleiten von HCl-Gas ausgefällt, abgesaugt und dann in einem Gemisch aus 30 ml Ethylenglykolmonomethylether und 10 ml Aceton unter Zugabe von konzentriertem Ammoniak und wenig Wasser unter Erhitzen gelöst. Bis zum Auftreten der ersten bleibenden Trübung wurde in der Siedehitze langsam mit Wasser versetzt, auf Zimmertemperatur abgekühlt und die ausgefallenen Kristalle abfiltriert. Das Rohprodukt wurde aus Ethylenglykolmonomethylether/Wasser (s.o.) umkristallisiert. Es wurden gelbe Kristalle vom Schmelzpunkt 225-229°C(unter Zersetzung) erhalten.

## Beispiel 2

Herstellung von 2,4-Diamino-5-(3,5-dimethoxy-4-[2-(4'-nitrophenyl-4-sulfonylphenylamino)ethoxy]benzyl)-pyrimidin (K 105)

a) 4-[N-(2-Brommethyl)-N-tosylamino]-4'-nitriphenylsulfid:
Anand et al, J. Sci. Industr. Res. 13B, 260-269 (1954) 40,05 g (0,1 Mol) 4-Tosylamino-4'-nitrodiphenylsulfid (Baker et al, J. Org. Chem. 15, 400 (1950) und 376 g (2,0 Mol) 1,2-Dibromethan wurden in 540 ml Ethanol nach der Zugabe von 100 mg Cu(I)J und einer Lösung von 8 g Kaliumhydroxid in 20 ml Wasser insgesamt 24 Stunden zum Sieden erhitzt. Nach 12 Stunden wurden erneut 8 g Kaliumhydroxid in 20 ml Wasser hinzugefügt und weiter zum Sieden erhitzt. Der Ansatz wurde auf ungefähr 150 ml einrotiert, die ausgefallene Substanz abfiltriert, mit Wasser gewaschen und getrocknet. Das Produkt wurde zur Reinigung in Methylenchlorid gelöst und mit Aktivkohle gereinigt oder aus Ethylacetat umkristallisiert. Es wurden in beiden Fällen weiß-gelbliche Kristalle mit einem Schmelzpunkt von 133-135°C erhalten.

b) 4-[N-(2-Bromethyl)-N-tosylamino]-4'-nitrodiphenylsulfon: (Anand et al., s.o.)
40,5 g (0,08 Mol) 4-N-(2-Bromethyl)-N-tosylamino-4'-nitrodiphenylsulfidwurden in einer Mischung von 94 ml konzentrierter Wasserstoffperoxidlösung und 500 ml Eisessig 4 Stunden auf dem Wasserbad bei 65°C gerührt. Das Endprodukt wurde durch Verdünnen mit 1000 ml Wasser und Kühlen im Eisbad vollständig ausgefällt, abfiltriert, mit Wasser gewaschen und gut getrocknet.

Es wurden weiße Kristalle mit einem Schmelzpunkt von 182-184°C erhalten.

c) 4-[N-(2-Bromethyl)amino]-4'-nitrodiphenylsulfon:
(Anand et al., s.o.)
16,2 g (0,03 Mol) getrocknete 4-[N-(2-Bromethyl)-N-tosylamino]-4'-nitrodiphenylsulfon wurden mit 32,5 ml konzentrierter Schwefelsäure versetzt und genau eine Stunde bei Zimmertemperatur gerührt. Danach wurde unter Rühren auf 300 ml Eis gegossen und zusätzlich mit 200 ml Wasser versetzt. Das ausgefallene gelbe Produkt wurde abfiltriert und mit Wasser gewaschen. Die Substanz wurde dann in 250 ml Aceton unter Erhitzen gelöst und mit verdünntem Ammoniak auf pH 9 eingestellt. Die Lösung wurde im Vakuum auf 100 ml einrotiert, erneut auf 500 ml Eis gegossen und die ausgefallene Substanz abfiltriert. Danach wurde aus Essigsäureethylester oder Aceton/Wasser umkristallisiert.

Es wurden gelbe Kristalle mit einem Schmelzpunkt von 164-167°C erhalten.

d) 2,4-Diamino-5-(3,5-dimethoxy-4-[2-(4'-nitrophenyl-4-sulfonylphenylamino)ethoxy]-benzyl)pyrimidin:
Es wurde aus 0,3 g (0,013 Mol) Natrium und 200 ml Ethylenglykolmonomethylether eine Alkoholatlösung hergestellt.

3,5 g (0,0126 Mol) 2,4-Diamino-5-(4-hydroxy-3,5-dimethoxybenzyl)pyrimidin gemäß Beispiel 1 wurden hinzugefügt und die Lösung so lange gerührt, bis das Phenol mit roter Farbe vollständig in Lösung gegangen war. Es wurden 0,1 g NaJ und 4,9 g (0,0127 Mol) 4-[N-(Bromethylamino]-4'-nitrodiphenylsulfon zugesetzt und die Lösung 4 Tage bei Zimmertemperatur gerührt. Der Ansatz wurde auf

500 ml Eis gegossen, die ausgefallene Substanz abfiltriert, getrocknet und in 250 ml Aceton unter erhitzen gelöst. Die Lösung wurde filtriert, auf ungefähr 5 ml im Vakuum einrotiert und über eine Säule (Durchmesser 6,5 cm, Länge 30 cm, Kieselgel (Partikelgröße entsprechend einer lichten Maschenweite von 0,21 bis 0,063 mm, 300 g) mit einem Fließmittelgemisch (Methylenchlorid (80 Teile)/n-Propanol (20 Teile)/Ammoniak konzentriert (1 Teil)) gereinigt.

Die Fraktionen der reinen Substanz wurden vereinigt und auf 20 ml einrotiert. Dabei fiel eine gelbe Substanz aus, die nach Abkühlen über Nacht abfiltriert wurde.

Es wurden gelbe Kristalle vom Schmelzpunkt 235-238°C (unter Zersetzung) erhalten.

## Beispiel 3

Herstellung von 2,4-Diamino-5-(3,5-dimethoxy-4-[3-(4'-nitrophenyl-4-sulfonylphenylamino)propoxy]-benzyl)-pyrimidin (K 122)

a) 4-[N-(3-Brompropyl)-N-tosylamino]-4'-nitrodiphenylsulfid:
40,05 g (0,1 Mol) 4-Tosylamino-4'-nitrodiphenylsulfid Gemäß Beispiel 3a und 150 ml (1,5 Mol) 1,3-Dibrompropan in 500 ml Ethanol wurden nach Zugabe von 0,1 g Cu(I)J zum Sieden erhitzt. Eine Lösung von 14 g Kaliumhydroxid in 30 ml Wasser wurde innerhalb von 8 Stunden langsam zur siedenden Lösung zugetropft und weitere 2 Stunden gekocht. Das Ethanol wurde unter vermindertem Druck, überschüssiges Dibrompropan mittels Wasserdampfdestillation erntfernt. Der verbliebene Rückstand wurde abgekühlt, in 300 ml Essigsäureethylester unter Erwärmen gelöst, mit 4 g Aktivkohle versetzt und filtriert. Die Lösung wurde mit 200 ml Ethanol versetzt, auf ungefähr die Hälfte einrotiert, kühl gestellt und die ausgefallenen Kristalle abfiltriert und in Essigsäureethylester umkristallisiert.

Es wurden Kristalle vom Schmelzpunkt 112-114°C erhalten.

b) 4-[N-(3-Brompropyl)-N-tosylamino]-4'-nitrodiphenylsulfon:
36,5 g (0,07 Mol) 4-[N-(3-Brompropyl)-N-tosylamino]-4'-nitrodiphenylsulfid wurden in 400 ml Eisessig unter Zusatz von 80 ml konzentrierter Wasserstoffperoxidlösung 4 Stunden bei 65°C gerührt. Die Lösung wurde wie in Beispiel 2b mit 900 ml Wasser verdünnt und das Endprodukt durch Kühlen im Eisbad vollständig ausgefällt. Es wurde in Methanol unter Zusatz von wenig Methylenchlorid umkristallisiert.

Es wurden weiße Kristalle vom Schmelzpunkt 153-155°C erhalten.

c) 4-[N-(3-Brompropyl)amino]-4'-nitrodiphenylsulfon:
10 g (0,018 Mol) 4-[N-Brompropyl)-N-tosylamino]-4'-nitrodiphenylsulfon wurden mit 20 ml konzentrierter Schwefelsäure versetzt und genau 60 Minuten bei Raumtemperatur gerührt. Der Ansatz wurde dann unter Rühren auf 500 ml Eis gegossen, abfiltriert, mit Wasser gewaschen und in 150-200 ml Aceton unter Erwärmen gelöst. Danach wurde die Lösung mit konzentriertem Ammoniak auf pH 9 eingestellt, filtriert, im Vakuum vollständig eingeengt und abgekühlt. Nachfolgend wurde in Methanol unter Zusatz von wenig Methylenchlorid umkristallisiert.

Es wurden gelbe Kristalle vom Schmelzpunkt 130-132°C erhalten.

d) 2,4-Diamino-5-(3,5-dimethoxy-4-[3-(4'-nitrophenyl-4-sulfonylphenylamino)propoxy]benzyl)-pyrimidin:
0,35 g (0,015 Mol) Natrium wurden in 100 ml Ethylenglykolmonomethylether gelöst, 4,2 g (0,015 Mol) 2,4-Diamino-5-(4-hydroxy-3,5-dimethoxybenzyl)-pyrimidin (vgl. Beispiel 1) hinzugefügt und gerührt, bis das Phenol vollständig in Lösung gegangen war. Dann wurde die Lösung mit 0,1 g NaJ und 6,2 g (0,155 Mol) 4-[N-(3-Brompropyl)amino]-4'-nitrodiphenylsulfon versetzt und 3 Tage bei Raumtemperatur gerührt.

Der Ansatz wurde mit 500 ml Eis versetzt, abfiltriert, mit Wasser gewaschen, in 300 bis 400 ml Aceton unter Erwärmen gelöst, erneut filtriert und im Vakuum auf 75 bis 100 ml einrotiert. Diese Lösung wurde über Nacht bei 5°C aufbewahrt und die ausgefallenen Kristalle abfiltriert. Nachfolgend wurde in Methanol unter Zusatz von wenig Methylenchlorid umkristallisiert.

Es wurden gelbe Kristalle mit einem Schmelzpunkt von 210-213°C erhalten.

## Beispiel 4

Herstellung von 2,4-Diamino-5-[4-methoxy-3-(4'-nitrophenyl-4-sulfonylphenyl)methoxybenzyl]-pyrimidin (K 127)

a) 3-Benzyloxy-4-methoxybenzaldehyd:
Aus 21,6 g (0,4 Mol) Natriumethylat, 60,9 g (0,4 Mol) 3-Hydroxy-4-methoxybenzaldehyd in 350 ml Ethylenglykolmonomethylether wurde eine Phenolatlösung hergestellt. Die Lösung wurde auf 100°C erhitzt, 51 g (0,4 Mol) Benzylchlorid hinzugefügt und 2 Stunden bei dieser Temperatur gerührt. Der

Ansatz wurde auf Raumtemperatur abgekühlt mit 600 ml Wasser versetzt und kühl gestellt. Das Rohprodukt wurde abfiltriert, aus Methanol umkristallisiert, getrocknet und weiterverarbeitet.

Es wurden weiße Kristalle vom Schmelzpunkt 62-63°C erhalten.

b) 3-Benzyloxy-4-methoxy-morpholinomethyliden-dihydrozimtsäure-nitril:

89,6 g (0,37 Mol) 3-Benzyloxy-4-methoxybenzaldehyd wurden unter Erwärmen auf 65°C in 500 ml Dimethylsulfoxid gelöst. Es wurden nacheinander 57 g (0,4 Mol) 3-Morpholinopropionsäurenitril und 4,5 g (0,083 Mol) Natriummethylat zugegeben, wonach die Temperatur auf über 70°C stieg. Man rührte weitere 15 Minuten bei 65°C, ließ dann auf Zimmertemperatur abkühlen, versetzte die Lösung mit einem Gemisch von 750 ml Isopropanol/Wasser (1:10) und kühlte über Nacht bei 5°C. Das Rohprodukt wurde mit Methylenchlorid extrahiert, die vereinigten Methylenchloridphasen über Natriumsulfat gut getrocknet und im Vakuum zur Trockne eingeengt. Das dickflüssige Rohprodukt wurde direkt weiterverarbeitet.

c) Anilinomethyliden-3-benzyloxy-4-methoxy-dihydrozimtsäure-nitril:

72,9 g (0,2 Mol) 3-Benzyloxy-4-methoxy-morpholinomethyliden-dihydrozimtsäure-nitril in 250 ml Isopropanol wurden mit 22,3 g (0,24 Mol) Anilin und 27 ml konz. Salzsäure versetzt und eine Stunde zum Sieden erhitzt. Man ließ auf Zimmertemperatur abkühlen, filtrierte die ausgefallene Substanz ab und wusch mit Wasser und Diethylether. Nachfolgend wurde aus iso-Propanol umkristallisiert.

Es wurden weiße Kristalle mit einem Schmelzpunkt von 148-151°C erhalten.

d) 2,4-Diamino-5-(3-benzyloxy-4-methoxybenzyl)-pyrimidin:

92,6 g (0,25 Mol) Anilinomethyliden-3-benzyloxy-4-methoxy-dihydrozimtsäure-nitril wurden mit 38,2 g (0,4 Mol) Guanidinhydrochlorid und 30,25 g (0,56 Mol) Natriummethylat in 600 ml Ethanol 22 Stunden lang zum Sieden erhitzt. Man ließ den Ansatz auf Raumtemperatur abkühlen, versetzte mit 20 ml konz. Ammoniak, filtrierte die ausgefallene Substanz ab und wusch mit Wasser.

Es wurden weiße Kristalle mit einem Schmelzpunkt von 210-213°C erhalten.

e) 2,4-Diamino-5-(3-hydroxy-4-methoxybenzyl)-pyrimidin:

20,2 g (0,06 Mol) 2,4-Diamino-5-(3-benzyloxy-4-methoxybenzyl)-pyrimidinwurden mit 4 g Palladium/Kohle 5% in 250 ml Ethylenglykolmonomethylether 45 Minuten bei 3 bar und 45°C hydriert. Die Lösung wurde filtriert, auf 600 ml Wasser gegossen, mit 10 ml konz. Ammoniak versetzt und die ausgefallene Substanz abfiltriert. Zur Umkristallisation wurde die Substanz in siedendem Wasser unter Zugabe von verdünnter Salzsäure gelöst, dann filtriert und mit konz. Ammoniak auf pH 8 eingestellt und langsam auf Raumtemperatur abgekühlt.

Es wurden weiße Kristalle mit einem Schmelzpunkt von 208-210°C erhalten.

f) 2,4-Diamino-5-[4-methoxy-3-(4'-nitrophenyl-4-sulfonylphenyl)methoxybenzyl]-pyrimidin:

6,65 g (0,027 Mol) 2,4-Diamino-5-(3-hydroxy-4-methoxybenzyl)-pyrimidin wurden unter Zusatz von 1,46 g (0,027 Mol) Natriummethylat in 30 ml Ethylenglykolmonomethylether gelöst. Die Lösung wurde innerhalb von 5 Stunden zu einer Suspension von 9,64 g (0,027 Mol) 4-Brommethyl-4'-nitrodiphenylsulfon (J. Chem. Soc. 22, 1508 (1957)) unter Rühren bei Raumtemperatur zugetropft. Man ließ über Nacht stehen, filtrierte die ausgefallene Substanz ab und wusch mit wenig Ethanol. Nachfolgend wurde aus Ethylenglykolmonomethylether umkristallisiert.

Es wurden gelbliche Kristalle mit einem Schmelzpunkt von 234-238°C erhalten.

## Beispiel 5

Herstellung von 2,4-Diamino-5-(4-methoxy-3-[2-(4'-nitrophenyl-4-sulfonylphenylamino)ethoxy]benzyl)-pyrimidin (K 132)

3 g (0,0122 Mol) 2,4-Diamino-5-(3-hydroxy-4-methoxybenzyl)pyrimidin (vgl. Beispiel 4) wurden in 15 ml Ethylenglykolmonomethylether unter Zusatz von 0,66 g (0,0122 Mol) Natriummethylat gelöst und innerhalb von 8 Stunden zu einer Suspension von 4,7 g (0,0122 Mol) 4-N-(2-Bromethyl)amino-4'-nitrodiphenylsulfon (vgl. Beispiel 2c) in 20 ml Ethylenglykolmonomethylether zugetropft und weitere 3 Tage bei Raumperatur gerührt. Die weitere Aufarbeitung erfolgte wie in Beispiel 4f. Es wurde aus Ethylenglykolmonomethylether umkristallisiert und gelbe Kristalle mit einem Schmelzpunkt von 178-182°C erhalten. Die Substanz kristallisiert laut Elementaranalyse mit 0,5 Mol $H_2O$.

## Beispiel 6

2,4-Diamino-5-(4-methoxy-3-[3-(4'-nitrophenyl-4-sulfonylphenylamino)propoxy]benzyl)-pyrimidin (K 135)

3 g (0,0122 Mol) 2,4-Diamino-5-(3-hydroxy-4-methoxybenzyl)pyrimidin (vgl. Beispiel 4) wurden in 15 ml

Ethylenglykolmonomethylether unter Zusatz von 0,66 g (0,0122 Mol) Natriummethylat gelöst und entsprechend Beispiel 4 zu einer Suspension von 4,9 g 4-[N-(3-Brompropyl)amino]-4'-nitrodiphenylsulfon in 20 ml Ethylenglycolmonomethylether zugetropft und 3 Tage bei Raumtemperatur gerührt. Die ausgefallene Substanz wurde abfiltriert und mit Ethanol und wenig Aceton gewaschen. Nachfolgend wurde aus Aceton oder Ethylenglykolmonomethylether umkristallisiert.

Es wurden gelbe Kristalle mit einem Schmelzpunkt von 131-134°C erhalten.

## Beispiel 7

Herstellung von 2,4-Diamino-5-[3-methoxy-4-(4'-nitrophenyl-4-sulfonylphenyl)methoxybenzyl]-pyrimidin (K 116)

a) 4-Benzyloxy-3-methoxybenzaldehyd:
Ausgehend von 4-Hydroxy-3-methoxybenzaldehyd wurde analog Beispiel 4a verfahren und weiße Kristalle mit einem Schmelzpunkt von 63-64°C erhalten.
b) 4-Benzyloxy-3-methoxy-morpholinomethyliden-dihydrozimtsäure-nitril:
Es wurde analog zu Beispiel 4b verfahren und das dickflüssige Rohprodukt weiterverarbeitet.
c) Anilinomethyliden-4-benzyloxy-3-methoxy-dihydrozimtsäure-nitril:
Die Umsetzung wurde analog zu Beispiel 4c vorgenommen und es wurden weiße Kristalle mit einem Schmelzpunkt von 119-121°C erhalten.
d) 2,4-Diamino-5-(4-benzyloxy-3-methoxybenzyl)-pyrimidin:
Es wurde analog zu Beispiel 4d verfahren und weiße Kristalle mit einem Schmelzpunkt von 161-163°C erhalten.
e) 2,4-Diamino-5-(4-hydroxy-3-methoxybenzyl)-pyrimidin:
Die Umsetzung wurde analog zu Beispiel 4e vorgenommen und es wurden weiße Kristalle mit einem Schmelzpunkt von 266-267°C erhalten.
f) 2,4-Diamino-5-[3-methoxy-4-(4'-nitrophenyl-4-sulfonylphenyl)methoxybenzyl]-pyrimidin:
Die Umsetzung wurde analog zu Beispiel 4f vorgenommen und es wurden gelbe Kristalle mit einem Schmelzpunkt von 210-213°C erhalten.

## Beispiel 8

Herstellung von 2,4-Diamino-5-(4-[3-(4'-aminophenyl-4-sulfonylphenylamino)propoxy]-3,5-dimethoxybenzyl)-pyrimidin (K 130)

a) 4-Amino-4'-[N-(3-brompropyl)amino)-diphenylsulfon
6 g (0,015 Mol) 4-[N-(3-Brompropyl)amino]-4'-nitrodiphenylsulfon wurden in 200 ml Methanol mit Raney Nickel (W 2) 60 Minuten bei 40°C und 4 bar hydriert. Die Lösung wurde filtriert, im Vakuum auf 80 ml einrotiert und kühl gestellt. Die ausgefallene Substanz wurde abfiltriert und aus Methanol oder Methanol/Wasser umkristallisiert. Es wurde eine weiße Substanz erhalten, die sich an der Luft verfärbte und einen Schmelzpunkt von 115-119°C aufwies.
b) 2,4-Diamino-5-(4-[3-(4'aminophenyl-4-sulfonylphenylamino)propoxy]-3,5-dimethoxybenzyl)-pyrimidin:
Aus 2,5 g 2,4-Diamino-5-(4-hydroxy-3,5-dimethoxybenzyl)-pyrimidin (vgl. Beispiel 1) und 0,21 g Natrium in 60 ml Ethylenglykolmonomethylether wurde eine Phenolatlösung, hergestellt, 3,4 g (0,092 Mol) 4-Amino-4'-N-(3-brompropyl)amino-diphenylsulfon und 50 mg Natriumjodid wurden hinzugefügt und diese Lösung 3 Tage bei Raumtemperatur gerührt. Die Lösung wurde auf 400 ml Eis gegossen, die ausgefallene Substanz abfiltriert und in 200 ml verdünnter Schwefelsäure unter Erwärmen auf 45°C gelöst, filtriert und unter Kühlen mit 3N Natronlauge auf pH 8 eingestellt. Die ausgefallene Substanz wurde abfiltriert und aus Ethylenglykolmonomethylether umkristallisiert oder aus verdünnter Schwefelsäure oder Salzsäure umgefällt. Es wurde eine weiße Substanz mit einem Schmelzpunkt von 203-205°C erhalten.

## Beispiel 9

2,4-Diamino-5-[4-(4'-aminophenyl-4-sulfonylphenylmethoxy)-3,5-dimethoxybenzyl]-pyrimidin (K 96)

1,8 g (0,0033 Mol) 2,4-Diamino-5-[3,5-dimethoxy-4-(4'-nitrophenyl-4-sulfonylphenyl)methoxybenzyl]-pyrimidin wurden in 40 ml Methanol bei 50°C und Normaldruck 3 Stunden mit Raney Nickel W2 (Org. Synth.

Coll. Vol. 3) hydriert.

Die Lösung wurde filtriert, zum Sieden erhitzt, mit Wasser in der Siedehitze bis zum Auftreten der ersten bleibenden Trübung versetzt, kühl gestellt und die ausgefallene Substanz abfiltriert. Es wurde aus Ethylenglykolmonomethylether/Wasser (s.o.) umkristallisiert und eine weiße Substanz mit einem Schmelzpunkt von 243-246°C erhalten.

**Beispiel 10**

2,4-Diamino-5-(4-[2-(4'-aminophenyl-4-sulfonylphenylamino)ethoxy]-3,5-dimethoxybenzyl)-pyrimidin (K 107)

1,2 g (0,002 Mol) 2,4-Diamino-5-(3,5-dimethoxy-4-[2-(4'-nitrophenyl-4-sulfonylphenylamino)ethoxy]-benzyl)pyrimidin wurden in 50 ml Ethylenglykolmonomethylether bei 50°C und einem Druck von 3 bar mit Raney Nickel W2 (s. Beispiel 9) 4 Stunden hydriert. Die Lösung wurde filtriert, mit 500 ml Wasser versetzt und im Eisbad abgekühlt. Die ausgefallene Substanz wurde abfiltriert und aus Ethylenglykolmonomethylether/Wasser umkristallisiert oder aus 3N Schwefelsäure (vgl. Beispiel 8b) umgefällt. Es wurde eine weiße Substanz mit einem Schmelzpunkt von 127-130°C erhalten. Die Substanz kristallisiert laut Elementaranalyse mit 1 Mol $H_2O$.

**Beispiel 11**

2,4-Diamino-5-(4-[3-(4'-aminophenyl-4-sulfonylphenylamino)propoxy]-3,5-dimethoxybenzyl)-pyrimidin (K 130)

3,6 g (0,006 Mol) 2,4-Diamino-5-(3,5-dimethoxy-4-[3-(4'-nitrophenyl-4-sulfonylphenyl)propoxy]benzyl)-pyrimidin wurden in einem Gemisch aus 40 ml Ethylenglykolmonomethylether und 160 ml Methanol 80 Minuten lang bei 50°C und 5 bar mit Raney Nickel hydriert.

Die Lösung wurde filtriert, mit 1000 ml Wasser versetzt, mit verdünnter Salzsäure angesäuert, erneut filtriert und mit verdünntem Ammoniak unter Eiskühlung auf pH 8 eingestellt. Die ausgefallene Substanz wurde abfiltriert und gut getrocknet.

Es wurde aus Ethylenglykolmonomethylether/Wasser umkristallisiert oder umgefällt (vgl. Beispiel 8b) und eine weiße Substanz mit einem Schmelzpunkt von 203-205°C erhalten.

**Beispiel 12**

2,4-Diamino-5-[3-(4'-aminophenyl-4-sulfonylphenylmethoxy)-4-methoxybenzyl]-pyrimidin (K 128)

Die Umsetzung erfolgte analog zu Beispiel 9. Es wurde aus Methanol oder Ethylenglykolmonomethylether/Wasser umkristallisiert und eine weiße Substanz mit einem Schmelzpunkt von 215-218°C erhalten.

**Beispiel 13**

2,4-Diamino-5-(3-[2-(4'-aminophenyl-4-sulfonylphenylamino)ethoxy]-4-methoxybenzyl)-pyrimidin (K 138)

Die Umsetzung erfolgte analog zu Beispiel 10. Es wurde aus Ethylenglykolmonomethylether/Wasser oder Methanol umkristallisiert oder umgefällt (vgl. Beispiel 8b) und eine weiße Substanz mit einem Schmelzpunkt von 148-151°C erhalten.

**Beispiel 14**

2,4-Diamino-5-(3-[3-(4'-aminophenyl-4-sulfonylphenylamino)propoxy]-4-methoxybenzyl)-pyrimidin (K 137)

Die Umsetzung erfolgte analog zu Beispiel 13. Es wurde eine weiße Substanz mit einem Schmelzpunkt von 207-210°C erhalten.

**Beispiel 15**

2,4-Diamino-5-[4-(4'-aminophenyl-4-sulfonylphenylmethoxy)-3-methoxybenzyl]-pyrimidin (K 120)

Die Umsetzung erfolgte analog zu Beispiel 10 und es wurde eine beige Substanz mit einem Schmelzpunkt von 220-222°Cerhalten.

**Beispiel 16**

2,4-Diamino-5(4-[3-(4'-methylphenyl-4-sulfonylphenylamino)propoxy]-3,5-dimethoxybenzyl)-pyrimidin (K 150)

a) 4-Amino-4'-methyldiphenylsulfid:
19,6 g (0,08 Mol) 4-Nitro-4'-methyldiphenylsulfid (J. Chem. Soc. 22, 1508 (1957)) wurden mit Raney Nickel W2 6 Stunden bei 45°C und 4 bar in 275 ml Methanol hydriert.

Die Lösung wurde danach vom Raney Nickel abfiltriert, mit 600 ml Wasser versetzt, mit konzentriertem Ammoniak auf pH 8 eingestellt und abfiltriert. Nachfolgend wurde aus Methanol umkristallisiert oder aus 3N Salzsäure umgefällt.

Es wurden weiße Kristalle vom Schmelzpunkt 182°C erhalten.

b) 4-Tosylamino-4'-methyldiphenylsulfid:
15 g (0,07 Mol) 4-Amino-4'-methyldiphenylsulfid wurden in 75 ml Pyridin mit 14,7 g (0,077 Mol) Toluolsulfochlorid versetzt und 2 Stunden lang bei Zimmertemperatur gerührt.

Die Lösung wurde danach mit einem Gemisch aus 100 ml Ethanol und 80 ml Wasser versetzt, im Eisbad gekühlt und die ausgefallene Substanz abfiltriert und mit viel Wasser gewaschen.

Die Umkristallisation erfolgte aus Ethanol und es wurden weiße Kristalle mit einem Schmelzpunkt von 147-149°C erhalten.

c) 4-N-(3-Bromopropyl)-N-tosylamino-4'-methyldiphenylsulfid:
Die Umsetzung erfolgte analog Beispiel 3a. Es wurde in Methanol umkristallisiert und es wurden weiße Kristalle mit einem Schmelzpunkt von 113-115°C erhalten.

d) 4-[N-(3-Brompropyl)-N-tosylamino]-4'-methyldiphenylsulfon:
Die Umsetzung erfolgte analog Beispiel 3b. Es wurde in Methanol umkristallisiert und es wurden weiße Kristalle mit einem Schmelzpunkt von 137-138°C erhalten.

e) 4-[N-(3-Brompropyl)amino]-4'-methyldiphenylsulfon:
Die Umsetzung erfolgte analog zu Beispiel 3c. Es wurde in Methanol umkristallisiert und es wurden weiße Kristalle mit einem Schmelzpunkt von 125-127°C erhalten.

f) 2,4-Diamino-5-(4-[3-(4'-methylphenyl-4-sulfonylphenylamino)propoxy]-3,5-dimethoxybenzyl)-pyrimidin:
Die Umsetzung erfolgte analog zu Beispiel 3d. Als Fließmittelgemisch diente Methylenchlorid (90 Teile)-/Methanol(10 Teile)/Ammoniak konz. (1 Teil). Die Fraktion wurde zur Trockene einrotiert und die zurückgebliebene weiße Substanz aus Methanol umkristallisiert.

Es wurden weiße Kristalle mit einem Schmelzpunkt von 157-158°C erhalten.

**Beispiel 17**

Nachweis der Hemmwirkung der erfindungsgemäßen Substanzen gegenüber Mycobakterien

Die minimale Hemmkonzentration der in Tabelle 1 angegebenen handelsüblichen und erfindungsgemäßen Substanzen wurde nach bekannten Standardverfahren ("Methoden zur Empfindlichkeitsprüfung von bakteriellen Krankheitserregern", DIN 58940, Teil 5) bestimmt.

Die Bestimmung wurde an den folgenden Bakterienkulturen vorgenommen:

1. Mycobakterium lufu (F. Portaels, Amls. Soc. belg. Méol. trop. 60, 381 (1980)), FIB 1-85 JS
2. Mycobakterium tuberculosis H 37 RV, FIB 2-85 JS
3. Mycobakterium marinum SN 1254, FIB 3-85 JS
4. Escherichia coli, FIB 4-85 JS

Die genannten Stämme sind unter den angegebenen Nummern in der Stammsammlung des Forschungsinstituts Borstel hinterlegt.

Zur Anzucht der Bakterienkulturen wurden Nährböden nach Gottsacker und Löwenstein Jensen sowie Nährmedien nach Lockemann oder Dubos-Davis verwendet (vgl. "Nachweisverfahren für Mycobakterien aus Untersuchungsmaterial, III, Nährbodenrezepte zur Kultur von Tuberkulosebakterien", 1978, Herausgeber: Deutsches Zentralkomitee zur Bekämpfung der Tuberculose, Poppenhusenstr. 14c, 2000 Hamburg 60).

Jeweils 5 x $10^{-3}$ bis 5 x $10^{-5}$ mg, bezogen auf Feuchtgewicht, der Mycobakterienstämme wurden in 2 ml Kulturflüssigkeit (Lockemann + 0,5 Gew./Vol.% Rinderserumalbumin-Fraktion 5, s.o.) bei 31°C mit abgestuften Konzentrationen des jeweiligen Hemmstoffs (Verdünnungsreihe) inkubiert. Die Ablesung der

Kulturröhrchen erfolgte je nach Bakterienstamm nach 8 bis 15 Tagen.

Die MHK ist diejenige Konzentration des Hemmstoffs in µMol/l, bei der keine Vermehrung der eingesäten Zellen festgestellt wird.

Die Ergebnisse sind in Tabelle 1 wiedergegeben. Sie zeigen, daß die erfindungsgemäßen Verbindungen eine gegenüber den Vergleichspräparaten bis um das 300fache gesteigerte antimycobakterielle Wirksamkeit aufweisen (vgl. MHK von Pyrimethamin und der erfindungsgemäßen Verbindung K 130 für Mycobakterium lufu).

Ferner wurde die MHK der Verbindung GH 310 wie oben angegeben, gegenüber einer Reihe von intrazellulären Mycobacterien bestimmt, die nach bekannten Verfahren ("Isolierung von Mycobacterien", DIN 58943, Teil 3, 1980) aus Patienten isoliert worden waren. Die Ergebnisse sind in Tabelle 2 wiedergegeben.

Tabelle 2

| Mycobacterium | MHK GH 310 µMol/l |
|---|---|
| M. smegmatis FIB 5-85 JS | 20 |
| M. avium FIB 6-85 JS | 5,2 |
| M. kansasii FIB 7-85 JS | 5,2 |
| M. simiae FIB 8-85 JS | 2,6 |

## Beispiel 8

Die halbmaximale Hemmkonzentration ($I_{50}$) der in Tabelle 1 angegebenen handelsüblichen und erfindungsgemäßen Substanzen wurde für Mycobakterium lufu (F. Portaels, s.o.) nach der Methode der Bakterienvermehrungskinetik (J.K. Seydel et al. Chemotherapie 29, 249 (1983)) bestimmt.

Zu diesem Zweck wurde eine frische 4 bis 6 Wochen alte Kultur von Mycobakterium lufu von einem Gottsacker-Nährboden in frisches modifiziertes Dubos-Davis-Medium + 0,2 Gew./Vol.% Rinderserumalbumin-Fraktion 5, (Nährbodenrezepte zur Kultur von Tuberculosebakterien - s.o.) überimpft. Um eine einheitliche Suspension zu erhalten, wurden die Bakterienzellen in 5 ml Medium in einem Homogenisator (Potter S) homogenisiert. Die Suspension wurde mit 15 ml Medium verdünnt und 4 Minuten lang bei 150 g zentrifugiert. Ein Teil des Überstandes wurde sofort zur Bestimmung der Zellzahl verwendet und der verbleibende Teil zum Ansetzen der Kultur. Zu diesem Zweck wurde die Suspension anschließend mit Medium so verdünnt, daß sie ca. $10^5$-Bakterienzellen/ml enthielt. Jeweils 50 ml Portionen der Kultur wurden dann in 300 ml Erlenmeyer-Kolben transferiert, die mit einem 2 cm Magnetrührer ausgerüstet waren, und der Hemmstoff zugesetzt. Die Hemmstoffkonzentrationen wurden so abgestuft, daß eine einwandfreie Berechnung der $I_{50}$-Werte möglich war (vgl. Abb. 1 und 2). Die Kulturen wurden bei 31°C gehalten und über einen Gesamtzeitraum von 330 Stunden im Abstand von 20 bis 30 Stunden Proben entnommen und die Gesamtzellzahl bestimmt.

Die Zellzahlbestimmung wurde durchgeführt, indem den Kulturen Proben von jeweils 1 ml entnommen und diese mit einer teilchenfreien wäßrigen Lösung mit einem Gehalt von 5% Kochsalz und 0,5% Formaldehyd so verdünnt wurden, daß Bakterienzahlen zwischen 500 und 90 000 erhalten wurden. Die verdünnten Lösungen wurden mit einem Coulter-Counter, Modell ZM, ausgerüstet mit einer 30 µm Kapillare gemessen. Es wurde die Bakterienzahl in jeweils 50 µl bei folgender Instrumenteneinstellung bestimmt:

| | |
|---|---|
| Verstärkung | 2 |
| Strom | 999 µA |
| Matching | 5 kOhm |
| unterer Schwellenwert | 7 |
| oberer Schwellenwert | offen (J.K. Seydel, E. Wempe und M. Rosenfeld Chemotherapie 29, 249 1983)). |

Die Ergebnisse der Vermehrungskinetiken von M. lufu in Gegenwart der Verbindungen K 107 bzw. K 130 sind in den Abbildungen 1 und 2, die $I_{50}$-Werte in Tabelle 1 wiedergegeben.

Tabelle 1

| Substanz | Bakterienkinetik $I_{50}$[μMol/l] | Minimale Hemmkonzentration (μ/Mol/l) | | | |
|---|---|---|---|---|---|
| | | M. lufu | M. tub. H37Rv | M. marin. SN1254 | E. coli |
| Trimethoprim | 95 | >110 | >110 | 28 | 1,4 |
| Brodimoprim | 45 | 80 | 94 | 18 | 1,4 |
| Tetroxoprim | 213 | - | 71 | - | 11,25 |
| Diaveridin | - | >123 | >123 | 46 | 8 |
| Pyrimethamin | - | 210 | 129 | - | - |
| K 95 | - | 58 | 29 | 29 | - |
| K 96 | - | 31 | >62 | >62 | >90 |
| K 107 | 1,37 | 1,8 | 33 | 3,6 | >45 |
| K 120 | - | 32,0 | 65 | 33 | >90 |
| K 122 | - | >58 | 3,4 | 13 | 11,25 |
| K 128 | 8,31 | 12,2 | 65 | 6,1 | >45 |
| K 130 | 1,66 | 0,7 | 4,0 | 6,2 | 32 |
| K 132 | - | 11,0 | 7,3 | 15 | 11 |
| K 135 | - | 10,6 | 7,1 | 14 | 11 |
| K 137 | - | 2,3 | 2,1 | 26 | >90 |
| K 138 | - | 3,8 | 11,5 | 7,7 | |
| K 150 | - | 28 | 28 | - | - |

**Beispiel 19**

Bestimmung der minimalen Hemmkonzentration (MHK) von Kombinationen der erfindungsgemäßen Verbindungen mit Diaminodiphenylsulfon (DDS)

Die MHK von Kombinationen der erfindungsgemäßen Verbindungen K 107 und K 130 mit DDS für Mycobakterium lufu wurde durch Schachbrett-Titrationen (s.o.) (vgl. Berenbaum, M.C., J. infect. Dis. 137, 122-130 (1978) und J.K. Seydel et al. s.o.) bestimmt.

Die Bakterien wurden unter den Bedingungen gemäß Beispiel 17 inkubiert.

Es wurde eine 2-dimensionale Verdünnungsreihe hergestellt, wobei die jeweiligen Komponenten in den Mengen gemäß Tabellen 3 und 4 vorlagen.

Die Summe der fraktionellen Hemmkonzentrationen (FII) wurde nach der Gleichung von C.W. Norden et al, J.Infect.-Dis. 140, 290 (1978) berechnet:

$$FII = \frac{\text{MKH der Verbindung A in Kombination}}{\text{MHK der Verbindung A allein}} + \frac{\text{MHK der Verbindung B in Kombination}}{\text{MHK der Verbindung B allein}}$$

Bei FII <1 liegt Synergismus vor während FII>>1 Antagonismus bedeutet.

Die Ergebnisse sind in den Tabellen 3 und 4 wiedergegeben. Es zeigte sich, daß nahezu jedes der gewählten Mischungsverhältnisse zu einem synergistischen Effekt führte.

Die FII für einige der Mischungsverhältnisse sind in den Tabellen 3 und 4 angegeben.

Tabelle 3

Mikroorganismus: M. lufu

← K 130 [µg/ml]

| DDS [µg/ml] ↑ | 0,4 | 0,36 | 0,32 | 0,28 | 0,24 | 0,20 | 0,16 | 0,12 | 0,08 | 0,04 | 0 |
|---|---|---|---|---|---|---|---|---|---|---|---|
| 0,2 | | | | | | | | | | | - |
| 0,018 | | | | | | | | | | | - |
| 0,016 | | | | | | | | | | | (+) |
| 0,014 | | | | | | | | | | | (+) |
| 0,012 | | | | | | | | | | | + |
| 0,01 | | | | | | | | | 1* | + | ++ |
| 0,008 | | | | | | | | | (+) | + | ++ |
| 0,006 | | | | | | | 2* | + | + | ++ | +++ |
| 0,004 | | | | 3* | | + | ++ | ++ | +++ | +++ | +++ |
| 0,002 | | | | (+) | ++ | +++ | +++ | +++ | +++ | +++ | +++ |
| 0 | | (+) | ++ | +++ | +++ | +++ | +++ | +++ | +++ | +++ | |

1* FII = 0 75
2* FII = 0 73
3* FII = 0 92
Dubos + 0,5% Albumin

+++ = Wachstum wie Kontrolle
++ = leichte Wachstumshemmung
+ = starke Wachstumshemmung
- = vollständige Wachstumshemmung

**Tabelle 4**

Mikroorganismus: M. lufu

DDS [µg/ml] ←

| K 107 [µg/ml] ↑ | 0,027 | 0,024 | 0,021 | 0,018 | 0,015 | 0,012 | 0,009 | 0,006 | 0,003 | 0,0 |
|---|---|---|---|---|---|---|---|---|---|---|
| 2,40 | | | | | | | | | | - |
| 2,16 | | | | | | | | | | - |
| 1,92 | | | | | | | | | | (+) |
| 1,68 | | | | | | | | | | (+) |
| 1,44 | | | | | | | | | | (+) |
| 1,20 | | | | | | | | | 1* | (+) |
| 0,96 | | | | | | | | | (+) | + |
| 0,72 | | | | | | | 2* | (+) | + | ++ |
| 0,48 | | | | | | (+) | + | + | ++ | +++ |
| 0,24 | | | 3* | (+) | + | + | ++ | ++ | +++ | +++ |
| 0,00 | - | (+) | (+) | + | + | ++ | +++ | +++ | +++ | |

1* FII = 0.68 2* FII = 0.45 3* FII = 0.98

Dubos + 0.5 % Albumin

+++ = Wachstum wie Kontrolle
++ = leichte Wachstumshemmung
+ = starke Wachstumshemmung
- = vollständige Wachstumshemmung

**Beispiel 20**

Kombinationspräparat in Tablettenform

Tabletten der folgenden Zusammensetzung wurden nach herkömmlichen Verfahren hergestellt:

| | |
|---|---|
| DDS | 100 mg |
| 2,4-Diamino-5-(4-decyloxy-3-methoxybenzyl)pyrimidin | 100 mg |
| Lactose | 150 mg |
| Primojel (Stärkederivat) | 3 mg |
| Povidone K 30 (Polyvinylpyrrolidon) | 4 mg |
| Magnesiumstearat | 3 mg |
| Gesamtgewicht | 360 mg |

## Patentansprüche

**1.** Substituierte 2,4-Diamino-5-benzylpyrimidine der allgemeinen Formel I

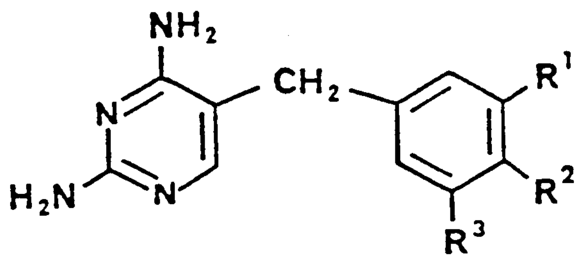

I ,

**dadurch gekennzeichnet**, daß einer der Substituenten $R^1$ bis $R^3$ eine 2',4'-substituierte Phenyl-4-sulfonylphenylaminoalkoxy-, Phenyl-4-sulfonylphenylaminoalkylthio-, Phenyl-4-sulfonylphenylalkoxy- oder Phenyl-4-sulfonylphenylalkylthiogruppe ist, wobei die Substituenten in 2',4'-Stellung gleich oder verschieden und Wasserstoff, Amino-, Alkylamino-, Dialkylamino-, Alkoxy-, Alkyl-, Nitro-, Alkylthio- und/oder Acetaminogruppen sind, und die beiden anderen der Substituenten $R^1$ bis $R^3$ gleich oder verschieden und Wasserstoff-, Alkoxy-, Alkylthio-, und/oder Alkylaminogruppen sind und die Alkylgruppen der beiden anderen Substituenten 1 bis 3 Kohlenstoffatome aufweisen.

**2.** 2,4-Diamino-5-[3,5-dimethoxy-4-(4'-nitrophenyl-4-sulfonylphenyl)methoxybenzyl]-pyrimidin (K 95).

**3.** 2,4-Diamino-5-(3,5-dimethoxy-4-[3-(4'-nitrophenyl-4-sulfonylphenylamino)propoxy]benzyl)-pyrimidin (K 122).

**4.** 2,4-Diamino-5-(4-methoxy-3-[2-(4'-nitrophenyl-4-sulfonylphenylamino)ethoxy]benzyl)-pyrimidin (K 132).

**5.** 2,4-Diamino-5-(4-methoxy-3-[3-(4'-nitrophenyl-4-sulfonylphenylamino)propoxy]benzyl)-pyrimidin (K 135).

**6.** 2,4-Diamino-5-[4-(4'-aminophenyl-4-sulfonylphenylmethoxy)-3,5-dimethoxybenzyl]-pyrimidin (K 96).

**7.** 2,4-Diamino-5-(4-[3-(4'-aminophenyl-4-sulfonylphenylamino)-propoxy]-3,5-dimethoxybenzyl)-pyrimidin (K 130).

**8.** 2,4-Diamino-5-[3-(4'-aminophenyl-4-sulfonylphenylmethoxy)-4-methoxybenzyl]-pyrimidin (K 128).

**9.** 2,4-Diamino-5-(3-[2-(4'-aminophenyl-4-sulfonylphenylamino)-ethoxy]-4-methoxybenzyl)-pyrimidin (K 138).

**10.** 2,4-Diamino-5-(4-[3-(4'-aminophenyl-4-sulfonylphenylamino)propoxy]-3,5-dimethoxybenzyl)-pyrimidin (K 137).

**11.** 2,4-Diamino-5-[4-(4'-aminophenyl-4-sulfonylphenylmethoxy)-3-methoxybenzyl]-pyrimidin (K 120).

**12.** 2,4-Diamino-5-(4-[3-(4'methylphenyl-4-sulfonylphenylamino)propoxy]-3,5-dimethoxybenzyl)-pyrimidin (K 150).

**13.** 2,4-Diamino-5-(4-[2-(4'-aminophenyl-4-sulfonylphenylamino)ethoxy]-3,5-dimethoxybenzyl)-pyrimidin (K 107).

**14.** 2,4-Diamino-5-(4-[2-(2'-methyl-4'aminophenyl-4-sulfonylphenylamino)ethoxy]-3,5-dimethoxybenzyl)-pyrimidin.

**15.** Physiologisch verträgliche Säureadditionssalze der Verbindungen nach den Ansprüchen 1 bis 14 mit anorganischen oder organischen Säuren.

**16.** Verfahren zur Herstellung der substituierten 2,4-Diamino-5-benzylpyrimidine nach den Ansprüchen 1 bis 15, **dadurch gekennzeichnet**, daß man entweder

a) eine Verbindung der allgemeinen Formel II

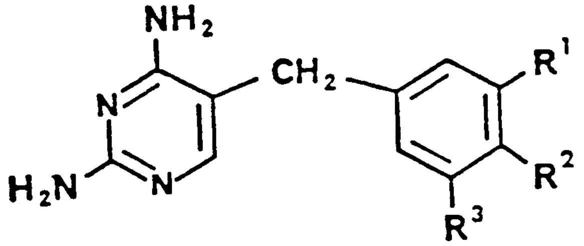

II ,

in der einer der Substituenten $R^1$ bis $R^3$ eine Hydroxyl- bzw. eine Mercaptogruppe ist und die beiden anderen der Substituenten $R^1$ bis $R^3$ gleich oder verschieden und Wasserstoff-, Alkoxy-, Alkylthio- und/oder Alkylaminogruppen sind, mit einer Verbindung der allgemeinen Formeln III

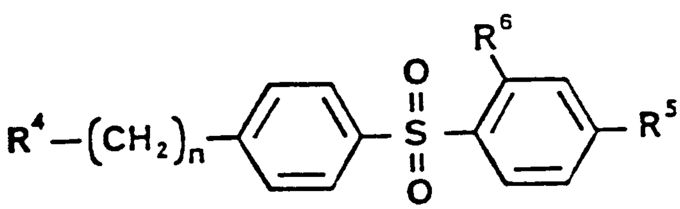

III

oder IV

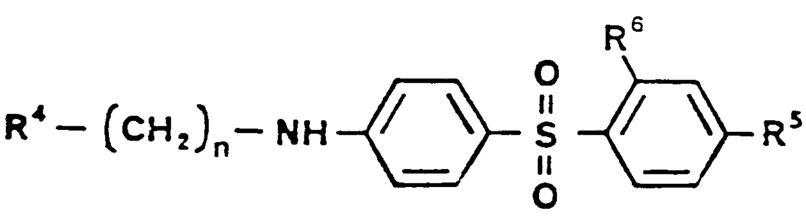

IV

verethert, in denen $R^4$ ein Halogenrest ist und $R^5$ und $R^6$ gleich oder verschieden und Wasserstoff, Amino-, Alkylamino-, Dialkylamino-, Alkoxy-, Alkyl-, Nitro-, Alkylthio- und/oder Acetaminogruppen sind, oder

b) eine Verbindung der allgemeinen Formel V

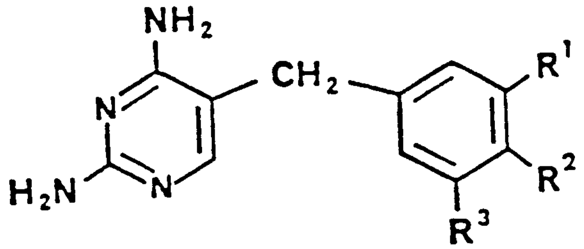

V

reduziert, in der einer der Substituenten $R^1$ bis $R^3$ eine 2',4'-substituierte Phenyl-4-sulfonylphenylaminoalkoxy-, Phenyl-4-sulfonylphenylaminoalkylthio-, Phenyl-4-sulfonylphenylalkoxy- oder Phenyl-4-sulfonylalkylthiogruppe mit endständiger Nitrogruppe ist, wobei die Substituenten in 2',4'-Stellung gleich oder verschieden und Wasserstoff, Amino-, Alkylamino-, Dialkylamino-, Alkoxy-, Alkyl-, Nitro-, Alkylthio- und/oder Acetamino-und die beiden anderen der Substituenten $R^1$ bis $R^3$ gleich oder verschieden Wasserstoff-, Alkoxy-, Alkylthio- und/oder Alkylaminogruppen sind.

**17.** Arzneimittel, **dadurch gekennzeichnet**, daß es eine oder mehrere der Verbindungen nach den Ansprüchen 1 bis 15 als antimikrobiellen Wirkstoff enthält.

**18.** Arzneimittel nach Anspruch 17, **dadurch gekennzeichnet**, daß es eine oder mehrere der Verbindungen nach den Ansprüchen 1 bis 15 in Kombination mit Hemmstoffen vom Typ der Diaminodiphenylsulfone, Ring-substituierten 4-Aminodiphenylsulfone oder Ring- und/oder Stickstoffsubstituierten Diaminodiphenylsulfone enthält.

**19.** Arzneimittel nach Anspruch 17, **dadurch gekennzeichnet**, daß es eine oder mehrere Verbindungen nach den Ansprüchen 1 bis 15 in Kombination mit antibakteriell wirkenden Sulfonamiden enthält.

**20.** Verwendung der Verbindungen nach den Ansprüchen 1 bis 15 sowie deren Kombinationen mit Hemmstoffen vom Typ der Diaminiodiphenylsulfone, Ring-substituierten 4-Aminodiphenylsulfone oder Ring- und/oder Stickstoff-substituierten Diaminodiphenylsulfone und/oder antibakteriell wirkenden Sulfonamiden zur Herstellung eines Arzneimittels zur Behandlung mikrobieller Infektionen und insbesondere mycobakterieller Infektionen.

## Claims

**1.** Substituted 2,4-diamino-5-benzylpyrimidines of the general formula I

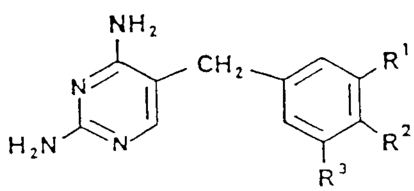

I

**characterized in that** one of the substituents $R^1$ to $R^3$

is a 2',4'-substituted phenyl-4-sulphonylphenyl aminoalkoxy, phenyl-4-sulphonylphenyl aminoalkylthio, phenyl-4-sulphonylphenylalkoxy or phenyl-4-sulphonylphenyl alkylthio group,

in which the substituents in 2'4' position are the same or different and are hydrogen, amino, alkylamino, dialkylamino, alkoxy, alkyl, nitro, alkylthio and/or acetamino groups,

and the two others of the substituents $R^1$ to $R^3$ are the same or different and are hydrogen, alkoxy, alkylthio and/or alkylamino groups and the alkyl groups of the two other substituents $R^1$ to $R^3$ display 1 to 3 carbon atoms.

**2.** 2,4-diamino-5-[3,5-dimethoxy-4-(4'-nitrophenyl-4-sulphonylphenyl)methoxybenzyl]-pyrimidine (K 95)

**3.** 2,4-diamino-5-[3,5-dimethoxy-4-[3-(4'-nitrophenyl-4-sulphonylphenylamino)propoxy]benzyl)-pyrimidine

(K 122)

**4.** 2,4-diamino-5-(4-methoxy-3-[2-(4'-nitrophenyl-4-sulphonylphenylamino)ethoxy]benzyl)-pyrimidine (K 132)

**5.** 2,4-diamino-5-[4-methoxy-3-[3-(4'-nitrophenyl-4-sulphonylphenylamino)propoxy]benzyl)-pyrimidine (K 135)

**6.** 2,4-diamino-5-[4-(4'-aminophenyl-4-sulphonylphenylmethoxy)-3,5-dimethoxybenzyl]-pyrimidine (K 96)

**7.** 2,4-diamino-5-(4-[3-(4-aminophenyl-4-sulphonylphenyl amino)-propoxy]-3,5-dimethoxybenzyl]-pyrimidine (K 130)

**8.** 2,4-diamino-5-[3-(4'-aminophenyl-4-sulphonylphenylmethoxy)-4-methoxyoxybenzyl]-pyrimidine (K 128)

**9.** 2,4-diamino-5-(3-[2-(4-aminophenyl-4-sulphonylphenylamino)-ethoxy]-4-methoxybenzyl)-pyrimidine (K 138)

**10.** 2,4-diamino-5-(4-[3-(4'-aminophenyl-4-sulphonylphenyl amino)propoxy]-3,5-dimethoxybenzyl)-pyrimidine (K137)

**11.** 2,4-diamino-5-[4-(4'-aminophenyl-4-sulphonylphenylmethoxy)-3-methoxybenzyl]-pyrimidine (K 120)

**12.** 2,4-diamino-5-(4-[3-(4'-methylphenyl-4-sulphonylphenylamino)-propoxy] -3,5-dimethoxybenzyl)-pyrimidine (K 150)

**13.** 2,4-diamino-5-(4-[2-(4'-aminophenyl-4-sulphonylphenylamino)ethoxy]-3,5-dimethoxybenzyl)-pyrimidine (K 107)

**14.** 2,4-diamino-5-(4-[2-(2'-methyl-4'-aminophenyl-4-sulphonylphenylamino) ethoxy]-3,5-dimethoxybenzyl)-pyrimidine.

**15.** Physiologically compatible acid addition salts of the compounds according to claims 1 to 14 with inorganic or organic acids.

**16.** Process for the preparation of the substituted 2,4-diamino-5-benzylpyrimidines according to claims 1 to 15, **characterized in that** either

a) a compound of the general formula II

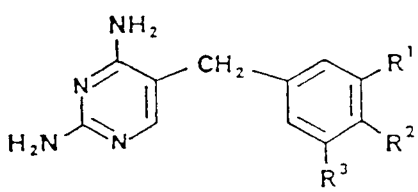

II

in which one of the substituents $R^1$ to $R^3$ is a hydroxyl or a mercapto group and the two others of the substituents $R^1$ to $R^3$ are the same or different and are hydrogen, alkoxy, alkylthio and/or alkylamino groups, is etherified with a compound of the general formula III

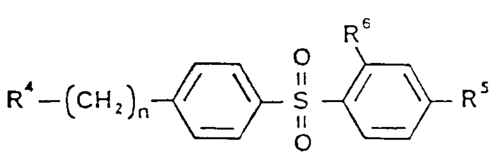

III

or IV

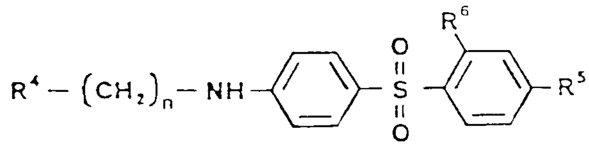

IV

in which $R^4$ is a halogen radical and $R^5$ and $R^6$ are the same or different and are hydrogen, amino, alkylamino, dialkylamino, alkoxy, alkyl, nitro, alkylthio and/or acetamino groups, or
b) a compound of the general formula V

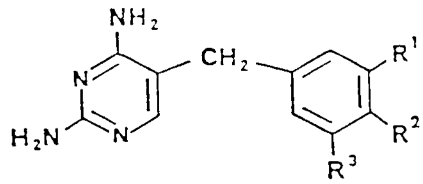

V

is reduced, in which one of the substituents $R^1$ to $R^3$ is a 2',4'-substituted phenyl-4-sulphonylphenyl aminoalkoxy, phenyl-4-sulphonylphenyl aminoalkylthio, phenyl-4-sulphonylphenyl-alkoxy or phenyl-4-sulphonyl alkylthio group with a terminal nitro group, the substituents in the 2',4'-position being the same or different and hydrogen, amino, alkylamino, dialkyl-amino, alkoxy, alkyl, nitro, alkylthio and/or acetamino groups and the two others of the substituents $R^1$ to $R^3$ being the same or different and hydrogen, alkoxy, alkylthio and/or alkylamino groups.

**17.** Medicament, **characterized in that** it contains one or more of the compounds according to claims 1 to 15 as antimicrobial active ingredient.

**18.** Medicament according to claim 17, **characterized in that** it contains one or more of the compounds according to claims 1 to 15 in combi-nation with inhibiting agents of the diaminodiphenylsulphones, ring-substituted 4-aminodiphenylsulphones or ring- and/or nitrogen-substituted diaminodiphenylsulphones types.

**19.** Medicament according to claim 17, **characterized in that** it contains one or more of the compounds according to claims 1 to 15 in combi-nation with antibacterially acting sulphonamides.

**20.** Use of the compounds according to claims 1 to 15 and combinations thereof with inhibiting agents of the diaminodiphenylsulphones, ring-substituted 4-aminodiphenylsulphones or ring- and/or nitrogen-substituted diaminodiphenylsulphones and/or antibacterially acting sulphonamides types for the preparation of a medicament for the treatment of microbial infections and in particular mycobacterial infections.

**Revendications**

**1.** 2,4-diamino-5-benzylpyrimidine substituée de formule générale I

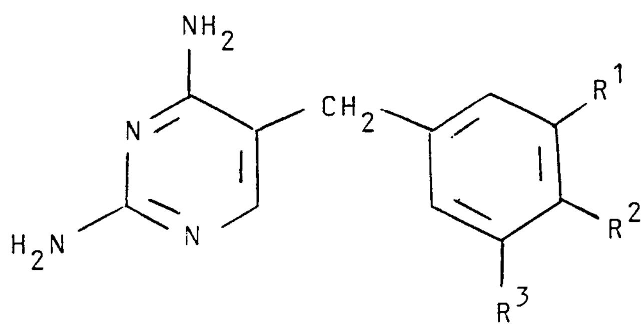

I ,

caractérisée en ce que l'un des substituants $R^1$ à $R^3$ est un groupe phényl-4-sulfonylphényl-aminoalcoxy, phényl-4-sulfonylphényl-aminoalkylthio, phényl-4-sulfonylphénylalcoxy ou phényl-4-sulfonylphénylalkylthio 2',4'-substitué,
les substituants en position 2',4' étant identiques ou différents et peuvent être hydrogène, un groupe amino, alkylamino, dialkylamino, alcoxy, alkyle, nitro, alkylthio et/ou acétamino,
et les deux autres substituants $R^1$ à $R^3$ peuvent être identiques ou différents et être hydrogène ou un groupe alcoxy, alkylthio et/ou alkylamino et les groupes alkyles des deux autres substituants présentent 1 à 3 atomes de carbone.

**2.** 2,4-diamino-5-[3,5-diméthoxy-4-(4'-nitrophényl-4-sulfonylphényl)méthoxybenzyl]-pyrimidine (K 95).

**3.** 2,4-diamino-5-(3,5-diméthoxy-4-[3-(4'-nitrophényl-4-sulfonylphénylamino)propoxy]benzyl)-pyrimidine (K 122).

**4.** 2,4-diamino-5-(4-méthoxy-3-[2-(4'-nitrophényl-4-sulfonylphénylamino)éthoxy]benzyl)-pyrimidine (K-132).

**5.** 2,4-diamino-5-(4-méthoxy-3-[3-(4'-nitrophényl-4-sulfonylphénylamino)propoxy]benzyl)-pyrimidine (K 135).

**6.** 2,4-diamino-5-[4-(4'-aminophényl-4-sulfonylphénylméthoxy)-3,5-diméthoxybenzyl]-pyrimidine (K 96).

**7.** 2,4-diamino-5-(4-[3-(4'-aminophényl-4-sulfonylphénylamino)-propoxy]-3,5-diméthoxybenzyl)-pyrimidine (K 130).

**8.** 2,4-diamino-5-[3-(4'-aminophényl-4-sulfonylphénylméthoxy)-4-méthoxybenzyl]pyrimidine (K 128).

**9.** 2,4-diamino-5-(3-[2-(4'-aminophényl-4-sulfonylphénylamino)-éthoxy]-4-méthoxybenzyl)-pyrimidine (K 138).

**10.** 2,4-diamino-5-(4-[3-(4'-aminophényl-4-sulfonylphénylamino)propoxy]-3,5-diméthoxybenzyl)-pyrimidine (K 137).

**11.** 2,4-diamino-5-[4-(4'-aminophényl-4-sulfonylphénylméthoxy)-3-méthoxybenzyl]-pyrimidine (K 120).

**12.** 2,4-diamino-5-(4-[3-(4'-méthylphényl-4-sulfonylphénylamino)propoxy]-3,5-diméthoxybenzyl)-pyrimidine (K 150).

**13.** 2,4-diamino-5-(4-[2-(4'-aminophényl-4-sulfonylphénylamino)éthoxy]-3,5-diméthoxybenzyl)-pyrimidine (K 107).

**14.** 2,4-diamino-5-(4-[2-(2'-méthyl-4'-aminophényl-4-sulfonylphénylamino)éthoxy]-3,5-diméthoxybenzyl)-pyrimidine.

**15.** Sels d'addition d'acide physiologiquement acceptables des composés selon l'une quelconque des revendications 1 à 14 avec des acides inorganiques ou organiques.

**16.** Procédé de préparation de la 2,4-diamino-5-benzylpyrimidine substituée selon les revendications 1 à 15, caractérisé en ce que, soit
a) on éthérifie un composé de la Formule générale II

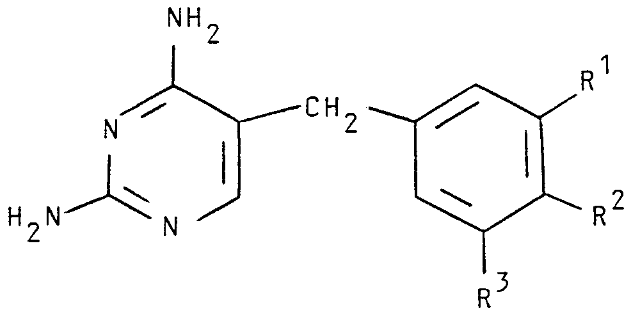

II ,

dans laquelle l'un des substituants $R^1$ à $R^3$ est un groupe hydroxyle ou mercapto et les deux autres substituants $R^1$ à $R^3$ sont identiques ou différents et sont hydrogène, ou bien un groupe alcoxy, alkylthio et/ou alkylamino, avec un composé de la Formule générale III

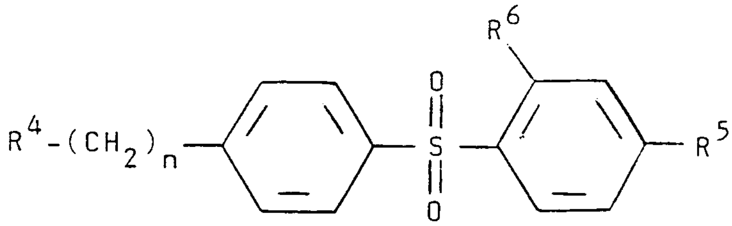

III

ou

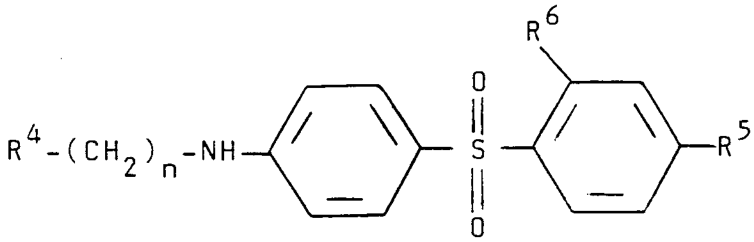

IV

où $R^4$ est un résidu d'halogène et $R^5$ et $R^6$ sont identiques ou différents et peuvent être hydrogène, ou un groupe amino, alkylamino, dialkylamino, alcoxy, alkyle, nitro, alkylthio et/ou acétamino, ou bien b) on réduit un composé de la Formule générale V

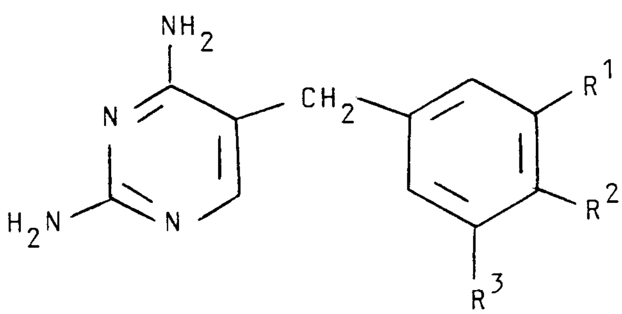

V

où l'un des substituants $R^1$ à $R^3$ est un groupe phényl-4-sulfonylphénylaminoalcoxy, phényl-4-sulfonylphénylaminoalkylthio, phényl-4-sulfonylphénylalcoxy ou phényl-4-sulfonylalkylthio 2',4'-substitué avec des groupes nitro en extrémité, les substituants en position 2',4' étant identiques ou

différents et pouvant être hydrogène, ou un groupe amino, alkylamino, dialkylamino, alcoxy, alkyle, nitro, alkylthio et/ou acétamino, les deux autres des substituants $R^1$ à $R^3$ étant identiques ou différents et pouvant être hydrogène, ou bien un groupe alcoxy, alkylthio et/ou alkylamino.

**17.** Médicament, caractérisé en ce qu'il contient, en tant qu'agent antimicrobien, un ou plusieurs des composés selon les revendications 1 à 15.

**18.** Médicament selon la revendication 17, caractérisé en ce qu'il contient un ou plusieurs des composés selon l'une quelconque des revendications 1 à 15 en combinaison avec des inhibiteurs du type diaminodiphénylsulfone, 4-aminodiphénylsulfone substituée dans le noyau ou bien diaminodiphénylsulfone substituée dans le noyau et/ou en azote.

**19.** Médicament selon la revendication 17, caractérisé en ce qu'il contient un ou plusieurs des composés selon l'une quelconque des revendications 1 à 15 en combinaison avec des sulfonamides présentant une action antibactérienne.

**20.** Utilisation des composés selon les revendications 1 à 15 ainsi que de leur combinaison avec des inhibiteurs du type diaminodiphénylsulfone, 4-aminodiphénylsulfone substituée dans le noyau ou bien diaminodiphénylsulfone substituée dans le noyau et/ou en azote et/ou des sulfonamides à action antibactérienne, pour la préparation d'un médicament pour le traitement des infections microbiennes et, en particulier des infections microbactériennes.

Abb. 1

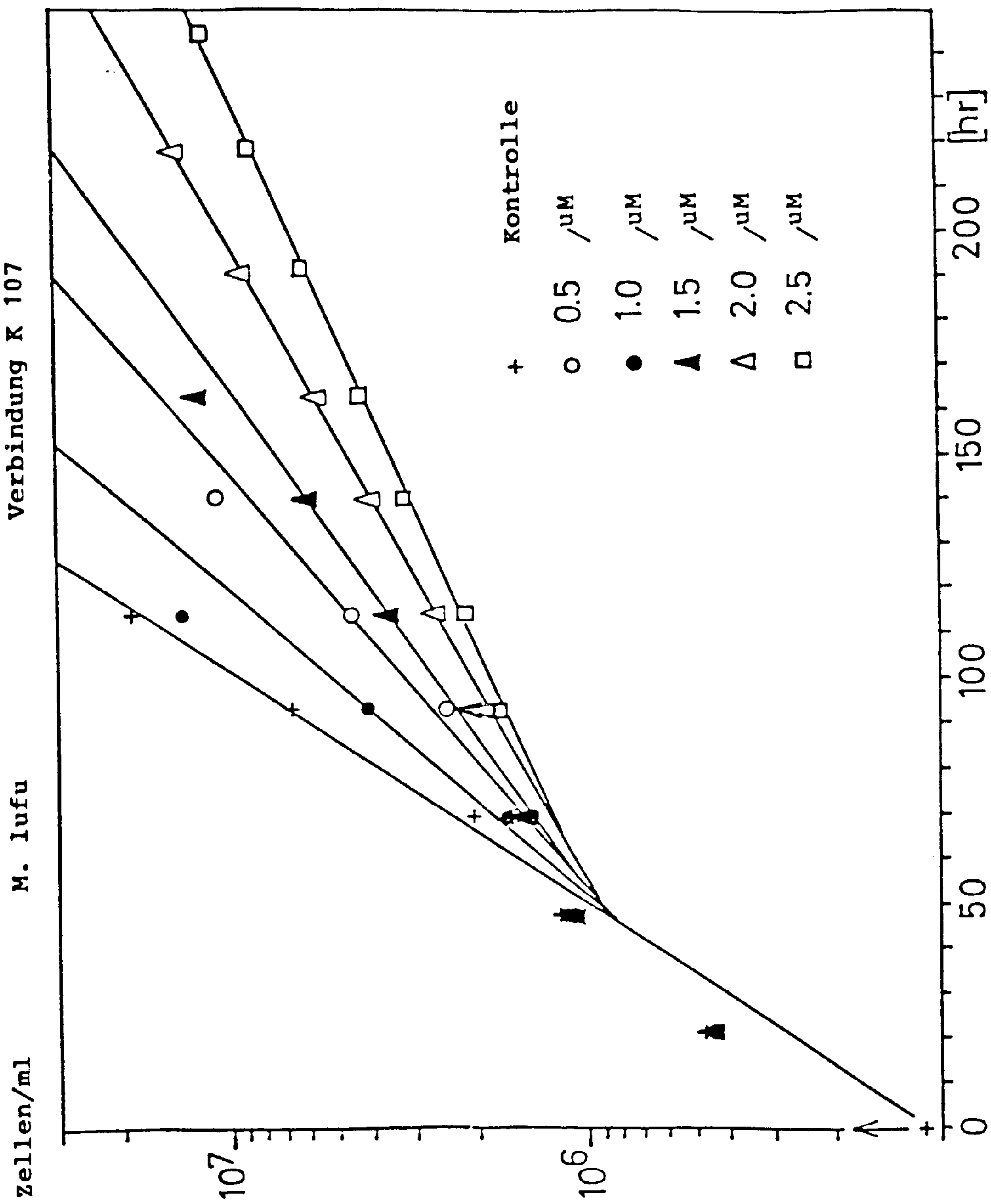
M. lufu
Verbindung K 107
Zellen/ml
$10^7$
$10^6$
0
50
100
150
200
[hr]
+ Kontrolle
○ 0.5 /uM
● 1.0 /uM
▲ 1.5 /uM
△ 2.0 /uM
□ 2.5 /uM

Abb. 2

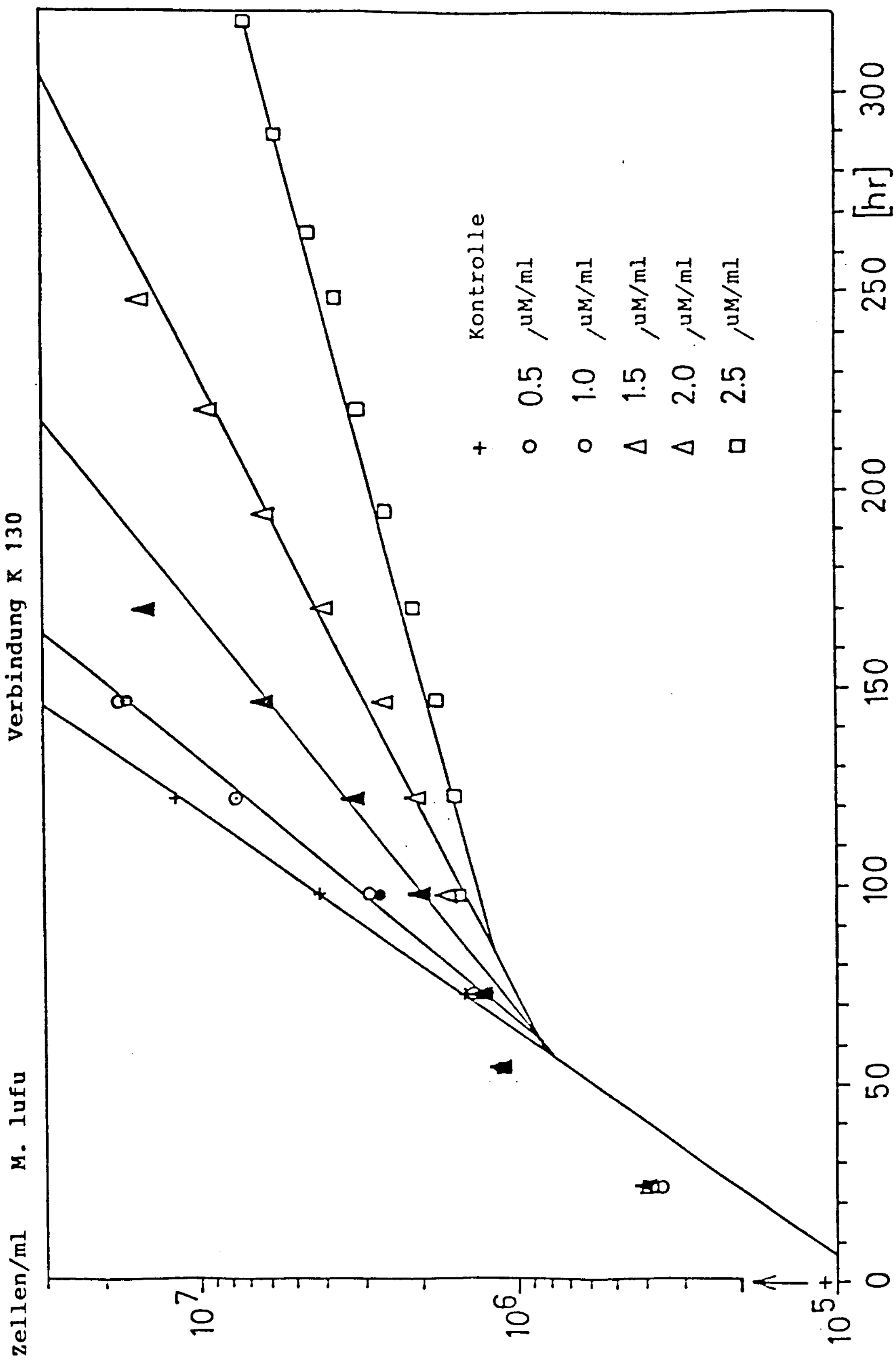

Verbindung K 130
M. lufu
Zellen/ml
$10^7$
$10^6$
$10^5$
0
50
100
150
200
250
300
[hr]
+ Kontrolle
o 0.5 uM/ml
o 1.0 uM/ml
Δ 1.5 uM/ml
Δ 2.0 uM/ml
□ 2.5 uM/ml